Europäisches Patentamt

European Patent Office

Office européen des brevets

(19)

(11) Veröffentlichungsnummer: **0 042 537**
**B1**

(12) **EUROPÄISCHE PATENTSCHRIFT**

(45) Veröffentlichungstag der Patentschrift:
29.08.84

(51) Int. Cl.³: **C 07 C 11/02**, C 07 C 5/25

(21) Anmeldenummer: **81104432.0**

(22) Anmeldetag: **10.06.81**

(54) Verfahren zur Stellungsisomerisierung von endständigen Doppelbindungen in Olefinen.

(30) Priorität: **19.06.80  DE 3022821**

(43) Veröffentlichungstag der Anmeldung:
**30.12.81 Patentblatt 81/52**

(45) Bekanntmachung des Hinweises auf die Patenterteilung:
**29.08.84 Patentblatt 84/35**

(84) Benannte Vertragsstaaten:
**DE**

(56) Entgegenhaltungen:
**DE - A - 2 449 298**
**SU - A - 172 308**
**US - A - 4 168 284**

(73) Patentinhaber: **EC ERDÖLCHEMIE GMBH,**
**Postfach 75 20 02, D-5000 Köln 71 (DE)**

(72) Erfinder: **Schöneberger, Helmut, Dr., Im Bergfeld 24,**
**D-6729 Woerth (DE)**
Erfinder: **Schleppinghoff, Bernhard, Dr.,**
**Adolf-Kolping-Strasse 5, D-4047 Dormagen 1 (DE)**
Erfinder: **Scheef, Hans-Volker, Goethestrasse 69,**
**D-4047 Dormagen 1 (DE)**

(74) Vertreter: **Mann, Volker, Dr. et al, c/o Bayer**
**Aktiengesellschaft Zentralbereich Patente Marken und**
**Lizenzen, D-5090 Leverkusen-Bayerwerk (DE)**

## Beschreibung

Die vorliegende Erfindung betrifft ein Verfahren zur Stellungsisomerisierung von endständigen Doppelbindungen in Olefinen in Gegenwart von stark sauren, wasserhaltigen Kationenaustauschern in der $H^+$-Form, wobei die endständigen Doppelbindungen an innenständige Positionen des Moleküls verschoben werden.

Die Lage der Doppelbindung von Olefinen ist von großer Bedeutung für ihre Motorklopffestigkeit und für die motorischen Eigenschaften des aus ihnen hergestellten Alkylatbenzins, das je nach seiner Struktur unterschiedliche Motorklopffestigkeit besitzt. Allgemein sind die Oktanzahlen der Olefine mit innenständiger Doppelbindung höher als die der Olefine mit endständiger Doppelbindung. Analog verhalten sich auch die aus diesen Olefinen gewonnenen Alkylate. So besitzt das Oktanalkylat von Buten-2 eine Research-Oktanzahl (ROZ) von 98,5, während das von Buten-1 eine ROZ von 92,5 hat (Oil and Gas Journal 8, 60 [1971]). Die Verschiebung endständiger Doppelbindungen in eine innenständige Lage des Olefins stellt daher einen wichtigen Schritt zur Gewinnung hochoktanigen Benzins dar.

Olefine mit innenständigen Doppelbindungen sind weiterhin als Einsatzstoffe für Disproportionierungen in Form der Metathese von Interesse. Zur metathetischen Darstellung von Neohexen muß das Trimethylpenten-1- zu Trimethylpenten-2 isomerisiert werden (US 3 660 516).

Es ist bereits bekannt, Buten-1 teilweise in Buten-2 in Gegenwart von Schwefelsäure umzuwandeln, wobei in homogener Phase in einem Bombengefäß bei etwa 77° C (US 2 334 998) oder in heterogener Phase in Gegenwart eines mit Schwefelsäure getränkten Silikagels bei etwa 93° C (US 2 386 983) gearbeitet wird.

Es ist weiterhin bekannt, Platin oder Palladium auf Aluminium oder Silikagel als Träger zur Umwandlung von Buten-1 in Buten-2 heranzuziehen, wobei entsprechend DE-OS 2 059 619 beispielsweise bei einer Temperatur von 500° C gearbeitet wird, während nach US 3 531 545 bei etwa 155° C und in Gegenwart von Wasserstoff gearbeitet wird, um die Oligomerenbildung einzuschränken.

Als ein weiterer Katalysator für die Umwandlung von Buten-1 zu Buten-2 ist ein gemischter Träger aus Aluminiumoxid und Silikagel, der mit Bortrifluorid getränkt wird, beschrieben (US 3 217 063), mit dem bei etwa 150° C und einem Druck von etwa 75 bar gearbeitet wird.

Von den beschriebenen Umwandlungskatalysatoren haben die Edelmetalle den Nachteil des hohen Preises, während Säure- und BF₃-Katalysatoren die Gefahr einer Verunreinigung des Umwandlungsproduktes mit sich bringen, die eine weitere Reinigung erforderlich macht. Die meisten der genannten Katalysatoren werden erst bei höherer Temperatur aktiv, obwohl für die gewünschte Einstellung des thermodynamischen Gleichgewichts niedrige Temperaturen zu bevorzugen sind.

Es ist ebenfalls bekannt, daß stark saure Kationenaustauscherharze in der $H^+$-Form, die für die Oligomerisierung von Olefinen eingesetzt werden, gleichzeitig auch endständige Doppelbindungen nach innen verschieben können (Hydrocarbon Processing 52 [4], 171 [1973]).

Aus US 4 168 284 ist es bekannt, höhere α-Olefine, wie 1-Dodecen, 1-Tetradecen, 1-Hexadecen und 1-Octadecen bei 110° C in Gegenwart eines wasserhaltigen stark sauren Kationenaustauschers zu Olefinen mit innenständiger Doppelbindung zu isomerisieren. Der Wassergehalt des Kationenaustauschers muß sich hierbei in den als sehr kritisch angesehenen Grenzen von 3—15 Gew.-% bewegen, um unerwünschte Nebenreaktionen weitgehend zu unterdrücken.

Aus SU 172 308 ist es bekannt, 3-Methylbuten-1 bei 150° C in 24stündiger chargenweiser Reaktion zu 63% in 2-Methyl-buten-2 umzuwandeln. Hierbei wird in Gegenwart eines mit Wasser gesättigten Kationenaustauscherharzes gearbeitet.

Nach der Beschreibung von DE-OS 2 449 298 wird das tertiäre Olefin 2-Methyl-penten-1 bei 40° C an einem getrockneten Kationenaustauscher zu etwa 85% in 2-Methylpenten-2 umgewandelt, wobei dem Einsatz-Methylpenten geringe Mengen 3-Hydroxy-3-methyl-pentan zugesetzt werden, das während der Reaktion Wasser abspaltet. Etwa 0,05 Gew.-% Wasser sollen die Polymerbildung wirksam unterdrücken, während bei Verwendung von zu viel Wasser der Katalysator entaktiviert wird.

Es wurde nun ein Verfahren zur Stellungsisomerisierung von endständigen Doppelbindungen in Olefinen mit 4 bis 8 Kohlenstoffatomen in Gegenwart eines stark sauren Kationenaustauscherharzes in der $H^+$-Form gefunden, das dadurch gekennzeichnet ist, daß der Ionenaustauscher einen Wassergehalt von 5 bis 60% seines Gesamtgewichts besitzt, der Wassergehalt des olefinhaltigen Einsatzproduktes im Bereich von 0 bis 95% der $H_2O$-Sättigungskonzentration gewählt wird und bei einer Temperatur von 50 bis 90° C gearbeitet wird.

Als stark saure Kationenaustauscherharze für das erfindungsgemäße Verfahren seien beispielsweise solche genannt, die auf einem mit Divinylbenzol vernetzten Polystyrol oder einem Phenol-Formaldehyd-Harz beruhen, das nachträglich durch Sulfonierung mit Sulfonsäuregruppen versehen worden ist und in der $H^+$-Form vorliegt. Solche Ionenaustauscher werden erfindungsgemäß mit einem Wassergehalt von 5 bis 60%, bevorzugt 15 bis 50%, ihres Gesamtgewichtes eingesetzt.

Als Olefine mit endständigen Doppelbindungen für das erfindungsgemäße Verfahren seien beispielsweise genannt: Buten-1, Penten-1, Hexen-1, Okten-1, 2,4,4-Trimethylpenten-1, 3,4,4-Trimethylpenten-1, 3,3,4-Trimethylpenten-1, 2,3,4-Trimethylpenten-1, 3,4-Dimethylhexen-1, 2,5-Dimethylhexen-1. Die genannten Olefine können im erfindungsgemäßen Verfahren entweder als reine Stoffe oder als Gemische mehrerer Olefine eingesetzt werden. Diese Olefine können weiterhin Ethylen und/oder Propylen sowie gesättigte Kohlenwasserstoffe und/oder Butadien oder Kohlenwasserstoffe mit einer Dreifachbindung enthalten. So kann es beispielsweise vorteilhaft sein, Destillationsschnitte, die in einer Raffinerie oder petrochemischen Anlage erhalten werden und die beispielsweise vorwiegend C$_4$- oder C$_5$-Kohlenwasserstoffe enthalten, der erfindungsgemäßen Stellungsisomerisierung der darin enthaltenen Olefine mit endständigen Doppelbindungen zu unterwerfen. Der Einsatz eines solchen, beispielhaft beschriebenen Gemisches ergibt vielfach den Vorteil, daß nach der durchgeführten Stellungsisomerisierung eine destillative Trennung in reine Stoffe, falls dies gewünscht wird, erleichtert ist.

Das olefinhaltige Einsatzprodukt für das erfindungsgemäße Verfahren kann wasserfrei sein oder auch Wasser enthalten, wobei der Wassergehalt im Bereich von 0 bis 95% der H$_2$O-Sättigungskonzentration des flüssigen Einsatzproduktes gewählt wird. Die bevorzugte H$_2$O-Konzentration liegt bei 20 bis 80% des Sättigungswertes. Der Wassergehalt des olefinischen Einsatzproduktes wird hierbei in Abstimmung mit dem Wassergehalt des Ionenaustauschers so gewählt, daß während des erfindungsgemäßen Verfahrens der Wassergehalt des Ionenaustauschers innerhalb des beschriebenen Bereiches bleibt. Dies kann jeweils durch einfache Vorversuche ermittelt werden. Der Wassergehalt des olefinischen Einsatzproduktes kann durch Zusatz von Wasser erreicht werden, sofern das olefinische Einsatzprodukt nicht bereits Wasser enthält. Das Wasser kann jedoch auch getrennt vom olefinischen Einsatzprodukt in das Reaktionsgemisch gegeben werden.

Die Produktmengengeschwindigkeit (WHSV = weight hourly space velocity) wird erfindungsgemäß bei 0,5 bis 5 kg Einsatzprodukt pro kg Katalysator pro Stunde, bevorzugt von 1,5 bis 3 kg Katalysator pro Stunde, eingestellt.

Das erfindungsgemäße Verfahren wird bei einer Temperatur von 50 bis 90° C, bevorzugt von 60 bis 80° C, und einem Druck von 0,2 bis 20 bar, bevorzugt 1,5 bis 12 bar, durchgeführt.

Das erfindungsgemäße Verfahren kann sowohl in der Flüssigphase als auch in der Gasphase durchgeführt werden, wobei der Katalysator sowohl im Festbett als auch als suspendierter Katalysator, beispielsweise in einem Schlaufenreaktor, angeordnet sein kann. Das erfindungsgemäße Verfahren erlaubt Umwandlungen von Olefinen mit endständigen Doppelbindungen zu den isomeren Olefinen mit mittelständigen Doppelbindungen zu etwa 90 bis 95% des theoretisch möglichen Umwandlungsgrades. Erfindungsgemäß können Olefine mit innenständigen Doppelbindungen und mindestens 4 C-Atomen hergestellt werden, wie Buten-2, Penten-2, Hexen-2, Hexen-3, 3,4-Dimethylhexen-2, 3,4,4-Trimethylpenten-2.

Für den Fall, daß das Einsatzmaterial für das erfindungsgemäße Verfahren 2 oder mehr Olefine mit entständigen Doppelbindungen enthält, werden im Reaktionsgemisch nach der Reaktion auch 2 oder mehr Olefine mit innenständigen Doppelbindungen enthalten. Diese können nach dem Fachmann bekannten Methoden, wie einer fraktionierten Destillation, einzeln gewonnen werden. Selbstverständlich ist es auch möglich, ein etwa erhaltenes Gemisch von 2 oder mehr Olefinen mit innenständigen Doppelbindungen, gegebenenfalls im Gemisch mit weiteren Olefinen und/oder Diolefinen und/oder Acetylenen und/oder Paraffinen ohne weitere Auftrennung weiterzuverwenden, falls dies, beispielsweise zur Herstellung von Alkylatbenzin, möglich ist.

Innerhalb der beschriebenen Reaktionsparameter wird eine Verminderung der Effektivität des Katalysators weder bei häufigem Einsatz in chargenweiser Anwendung noch bei länger dauerndem Einsatz im kontinuierlichen Verfahren beobachtet. Weiterhin wird eine Oligomerisierung der olefinischen Einsatzprodukte nur in einem untergeordneten Ausmaß bis höchstens 2 Gew.-% Oligomerisierung der eingesetzten Olefine beobachtet. Das erfindungsgemäße Verfahren benötigt einen preiswerten Katalysator ohne korrosive Eigenschaften, so daß als Reaktormaterial normale Kohlenstoffstähle verwendet werden können. Im Gegensatz zu einigen Isomerisierungsverfahren des Standes der Technik wird im erfindungsgemäßen Verfahren kein Wasserstoff benötigt.

### Beispiel 1

Über einen mantelbeheizten Stahlreaktor mit einem freien Durchmesser von 30 mm und einem Kontakt-Volumen von 500 ml wurde ein Buten-1 enthaltender KW-Strom mit der in der Tabelle 1 angegebenen Zusammensetzung geleitet. Der eingesetzte Kontakt war ein makroporöser, stark saurer Kationenaustauscher auf der Basis eines sulfongruppenhaltigen, mit Divinylbenzol vernetzten Polystyrols (SPC 118 von Fa. Bayer AG) in der H$^+$-Form. Der Wassergehalt des Austauschers war auf 21 Gew.-% eingestellt.

Als Isomerisierungsbedingungen wurden gewählt:

T = 65°C; p = 2 bar; WHSV (= weight hourly space velocity = stündlicher Raum-Mengen-Durchsatz) =

$$2 \frac{\text{g Einsatzprod.}}{\text{g Kat.} \cdot \text{h}};$$

t = 90 h

Der H$_2$O-Gehalt im Einsatz-C$_4$ betrug 20 ppm.

Die Isomerisierungsergebnisse sind in der Tabelle dargestellt.

Tabelle 1

| Produkt-Verteilung | Einsatz (Gew.-%) | nach Isomeris. (Gew.-%) |
|---|---|---|
| Buten-1 | 20,2 | 4,7 |
| Buten-2 | 53,5 | 64,8 |
| Rest C$_4$-Kohlenwasserstoffe | 25,7 | 28,7 |
| Summe C$_8$-Kohlenwasserstoffe | 0,6 | 1,8 |

Der Umsatz an Buten-1 betrug 76,7%.

### Beispiel 2

Im Beispiel 2 wurde die Apparatur von Beispiel 1 übernommen, der Katalysator entsprach dem aus Beispiel 1. Das Einsatzprodukt setzte sich etwas anders zusammen. Die Reaktionsparameter wurden geändert wie folgt:

$$T = 77°C; p = 15 \text{ bar}; WHSV = 3, t = 20 \text{ h}$$

Die Isomerisierungsergebnisse sind in der Tabelle 2 aufgeführt. Der H$_2$O-Gehalt im Einsatz-C$_4$ betrug 20 ppm.

Tabelle 2

| Produkt-Verteilung | Einsatz (Gew.-%) | nach Isomeris. (Gew.-%) |
|---|---|---|
| Buten-1 | 19,6 | 4,7 |
| Buten-2 | 58,9 | 72,9 |
| Rest C$_4$-Kohlenwasserstoffe | 21,4 | 21,5 |
| Summe C$_8$-Kohlenwasserstoffe | 0,1 | 0,9 |

Der Umsatz an Buten-1 betrug 76,0%.

### Beispiel 3

Die Apparatur von Beispiel 1 wurde übernommen. Der eingesetzte stark saure, makroporöse Ionenaustauscher in der H$^+$-Form wurde auf einen Wassergehalt von 60 Gew.-% eingestellt. Als Isomerisierungsbedingungen wurden gewählt:

$$T = 65°C; p = 2 \text{ bar}; WHSV = 2; t = 24 \text{ h}$$

Das Einsatzprodukt enthielt 20 ppm H$_2$O. Ergebnisse in Tabelle 3.

Tabelle 3

| Produkt-Verteilung | Einsatz (Gew.-%) | nach Isomeris. (Gew.-%) |
|---|---|---|
| Buten-1 | 20,2 | 5,2 |
| Buten-2 | 53,5 | 64,2 |
| Rest C$_4$-Kohlenwasserstoffe | 25,7 | 28,6 |
| Summe C$_8$-Kohlenwasserstoffe | 0,6 | 2,0 |

Der Umsatz an Buten-1 betrug 74,3%.

### Beispiel 4

Die Versuchsbedingungen waren mit denen von Beispiel 3 identisch, zusätzlich wurden dem Einsatzprodukt jedoch 500 ppm H$_2$O zugemischt. Ergebnisse in Tabelle 4.

Tabelle 4

| Produkt-Verteilung | Einsatz (Gew.-%) | nach Isomeris. (Gew.-%) |
|---|---|---|
| Buten-1 | 20,2 | 11,3 |
| Buten-2 | 53,5 | 61,3 |
| Rest C$_4$-Kohlenwasserstoffe | 25,7 | 26,2 |
| Summe C$_8$-Kohlenwasserstoffe | 0,6 | 1,2 |

Der Umsatz an Buten-1 betrug 44%.

### Beispiel 5

(Vergleichsbeispiel zeigt, daß bei geringem H$_2$O-Gehalt der Oligomerenanteil steigt)

Die Versuchsbedingungen von Versuch 3 wurden übernommen. Geändert wurde der Kationen-Austauscher. Zum Einsatz kam ein stark saurer, makroporöser Kationenaustauscher (Bayer SPC 118 H) mit einem Restwassergehalt von 0,5 Gew.-%.

Als Isomerisierungsbedingungen wurden gewählt:

$$T = 65°C; p = 2 \text{ bar}; WHSV = 2; t = 6 \text{ h}$$

Das Einsatz-$C_4$ enthielt 20 ppm $H_2O$.

Als Versuchsergebnis wurde gefunden (Tabelle 5):

Tabelle 5

| Produkt-Verteilung | Einsatz (Gew.-%) | nach Isomeris. (Gew.-%) |
|---|---|---|
| Buten-1 | 20,2 | 5,8 |
| Buten-2 | 53,5 | 65,3 |
| Rest $C_4$-Kohlenwasserstoffe | 25,7 | 24,9 |
| Summe $C_8$-Kohlenwasserstoffe | 0,6 | 3,4 |
| Höhere Kohlenwasserstoffe | 0,1 | 0,6 |

Der Umsatz an Buten-1 betrug 71,2%, teilweise hervorgerufen durch die Oligomerisierung des Buten-1.

## Patentansprüche

1. Verfahren zur Stellungsisomerisierung von endständigen Doppelbindungen in Olefinen mit 4 bis 8 Kohlenstoffatomen in Gegenwart eines stark sauren Kationenaustauscherharzes in der $H^+$-Form, dadurch gekennzeichnet, daß der Ionenaustauscher einen Wassergehalt von 5 bis 60% seines Gesamtgewichts besitzt, der Wassergehalt des olefinischen Einsatzproduktes im Bereich von 0 bis 95% der $H_2O$-Sättigungskonzentration gewählt wird und bei einer Temperatur von 50 bis 90°C gearbeitet wird.

2. Verfahren nach Anspruch 1, dadurch gekennzeichnet, daß als olefinisches Einsatzprodukt ein Gemisch eines Olefins mit endständiger Doppelbindung verwendet wird, das gegebenenfalls eines oder mehrere andere Olefine mit endständiger Doppelbindung, ein oder mehrere Diolefine, ein oder mehrere Acetylene sowie paraffinische Kohlenwasserstoffe enthält.

3. Verfahren nach Ansprüchen 1 und 2, dadurch gekennzeichnet, daß ein olefinisches Einsatzprodukt mit einem Wassergehalt von 20 bis 80% des Sättigungswertes verwendet wird.

4. Verfahren nach Ansprüchen 1 bis 3, dadurch gekennzeichnet, daß als olefinische Einsatzprodukte olefinhaltige Destillationsschnitte mit vorwiegend $C_4$- oder $C_5$-Kohlenwasserstoffen verwendet werden.

## Claims

1. Process for the positional isomerisation of terminal double bonds in olefines with 4 to 8 carbon atoms in the presence of a strongly acid cation exchanger resin in the $H^+$ form, characterised in that the ion exchanger has a water content of 5 to 60% of its total weight, the water content chosen for the olefinic starting material is in the range from 0 to 95% of the $H_2O$-saturation concentration, and the process is carried out at a temperature of 50 to 90°C.

2. Process according to Claim 1, characterised in that a mixture of an olefine with a terminal double bond, which optionally contains one or more other olefines with a terminal double bond, one or more diolefines, one or more acetylenes and paraffinic hydrocarbons, is used as the olefinic starting material.

3. Process according to Claims 1 and 2, characterised in that an olefinic starting material with a water content of 20 to 80% of the saturation value is used.

4. Process according to Claims 1 to 3, characterised in that olefinic distillation cuts containing predominantly $C_4 - C_5$-hydrocarbons are used sa the olefinic starting materials.

## Revendications

1. Procédé pour l'isomérisation de position de doubles liaisons terminales dans des oléfines ayant 4 à 8 atomes de carbone en présence d'une résine échangeuse de cations fortement acide sous la forme $H^+$, caractérisé en ce que l'échangeur d'ions possède une teneur en eau de 5 à 60% de son poids total, la teneur en eau de la charge oléfinique est choisie dans le domaine de 0 à 95% de la concentration à saturation en $H_2O$ et on opère à une température de 50 à 90°C.

2. Procédé selon la revendication 1, caractérisé en ce qu'on utilise comme charge oléfinique un mélange d'une oléfine à double liaison terminale, qui contient éventuellement une ou plusieurs autres oléfines à double liaison terminale, une ou plusieurs dioléfines, un ou plusieurs acétylènes ainsi que des hydrocarbures paraffiniques.

3. Procédé selon les revendications 1 et 2, caractérisé en ce que l'on utilise une charge oléfinique ayant une teneur en eau de 20 à 80% de la valeur à saturation.

4. Procédé selon les revendications 1 à 3, caractérisé en ce que l'on utilise comme charges oléfiniques des coupes de distillation contenant des oléfines consistant principalement en hydrocarbures en $C_4$ ou en $C_5$.